# FASCICULE DE BREVET EUROPEEN

(11) **EP 1 198 211 B1**
(45) Date de publication et mention de la délivrance du brevet: **07.03.2007**
(21) Numéro de dépôt: 00956570.6
(22) Date de dépôt: 28.07.2000
(51) Int. Cl.: A61F 5/00, A61B 17/12

(54) **DISPOSITIF TELECOMMANDE DE BANDE GASTRIQUE**
FERNSTEUERUNGSVORRICHTUNG FÜR MAGENBAND
GASTRIC BAND REMOTE CONTROL DEVICE

(30) Priorité: 05.08.1999 FR 9910206
(43) Date de publication de la demande: 24.04.2002
(73) Titulaire: Europlak, 83130 La Garde (FR)
(72) Inventeur: Cancel, Richard, 83130 La Garde (FR); Wallace, Richard, 83590 Gonfaron (FR); Sassi, Gérard, 83200 Toulon (FR)
(74) Mandataire: Breese, Pierre
(86) Numéro de dépôt international: PCT/FR2000/002191
(87) Numéro de publication internationale: WO 2001/010359

(56) Documents cités:
- EP-A- 0 611 561
- EP-A- 0 876 808
- WO-A-00/09048
- WO-A-00/15158
- WO-A-96/01597
- GB-A- 1 174 814
- US-A- 3 840 018

## Description

La présente invention concerne une bande de cerclage gastrique.

De tels dispositifs sont destinés notamment à réduire le poids des patients souffrant d'obésité sévère, en limitant la consommation de nourriture. Il se présente généralement sous la forme d'une boucle coulissante permettant une mise en place laparoscopique autour de l'estomac, avec la formation d'une petite poche gastrique et d'un rétrécissement.

Le brevet européen EP611561 décrit une bande gastrique ajustable pour laparoscopie comprenant :
- un élément de bande incluant une portion de bande pour encercler l'estomac de façon à former l'ouverture de stoma restreinte, la portion de bande ayant une extrémité libre, où l'élément de bande comporte en outre une section d'expansion fixée à la portion de bande et comprenant un élément oblong gonflable s'étendant complètement le long de la portion de bande de telle sorte que la section d'expansion encercle complètement l'estomac lorsque le dispositif est en place formant l'ouverture de stoma restreinte ; et étant caractérise par
- un premier moyen de fixation d'inter verrouillage disposé à une première position prédéterminée fixe sur la portion de bande à proximité de ladite extrémité libre, et un deuxième moyen de fixation d'interverrouillage disposé à une deuxième position prédéterminée fixe située dans une portion intermédiaire de l'élément de bande pour l'interverrouillage avec le premier moyen de fixation d'interverrouillage pour permettre la fixation de ladite extrémité libre de la portion de bande à la portion intermédiaire de celle-ci à ladite deuxième position de telle sorte que la portion de bande forme un cercle d'un diamètre prédéterminé fixe qui est prédéterminé par les emplacements des premières et deuxième positions fixes sur l'élément de bande.

D'autres brevets de l'art antérieur décrivent de tels dispositifs.

Le brevet US5549621 décrit un dispositif comportant deux éléments pivotant l'un par rapport à l'autre pour refermer la paroi de la poche gastrique.

Le brevet américain US4696288 décrit un dispositif comprenant un ballon toroïdal pouvant être engagé dans l'estomac.

Le brevet suédois SE8503144 décrit une prothèse gastrique pour le traitement de l'obésité.

Le brevet américain US4592339 décrit un dispositif pour rétrécir le stoma par une bande expansible.

La demande internationale de brevet WO 96/01597 décrit un appareil d'occlusion de conduits corporels, ledit dispositif comprenant un corps (45) prolongé par un élément de bande de cerclage (60, 63) incluant une portion de bande pour encercler le conduit, la portion de bande ayant une extrémité libre, ledit corps (45) contenant un moyen motorisé et la bande de cerclage étant entraînée par un moyen télécommandé.

Dans ce dispositif, la bande de cerclage (60, 63) comporte une partie non compressible (60) et vient se refermer sur elle-même.

Le document EP0876808 décrit une bande gastrique, comprenant les caractéristiques du préambule de la revendication de la présente demande.

L'invention vise à améliorer les bandes gastriques de façon à permettre un réglage de la boucle de serrage après sa mise en place, sans intervention chirurgicale. Le but est notamment de permettre l'évolution du traitement en fonction des réactions du patient.

A cet effet, l'invention concerne selon son acception la plus générale un dispositif de bande gastrique pour former une ouverture restreinte de stoma dans l'estomac de façon à restreindre l'admission de la nourriture vers une partie digestive inférieure de celui-ci comprenant un élément de bande de cerclage incluant une portion de bande pour encercler l'estomac de façon à former l'ouverture de stoma restreinte, la portion de bande ayant une extrémité libre, dans lequel la bande de cerclage est télécommandée.

Elle est formée en un matériau creux à l'intérieur duquel coulisse un tendeur dont l'une des extrémités est solidaire de l'extrémité libre de la bande de cerclage et dont l'autre extrémité est entraînée longitudinalement par un moyen motorisé comportant un moteur électrique télécommandé.

De préférence, le moyen motorisé comporte une vis sans fin entraînant l'extrémité du tendeur.

Avantageusement, le moyen motorisé comporte un moteur électrique télécommandé.

Selon une variante particulière, le tendeur est formé par un ruban en un matériau élastique.

Selon un mode de réalisation particulier, la bande présente une extrémité pourvue d'un moyen de coopération avec le corps contenant le moyen motorisé.

Selon une variante, le moyen de coopération est formé par un système d'ancrage dans une cavité complémentaire prévue sur le corps contenant le moyen motorisé.

L'invention sera mieux comprise à la lecture de la description qui suit, se référant à un exemple non limitatif de réalisation et aux figures annexées où :

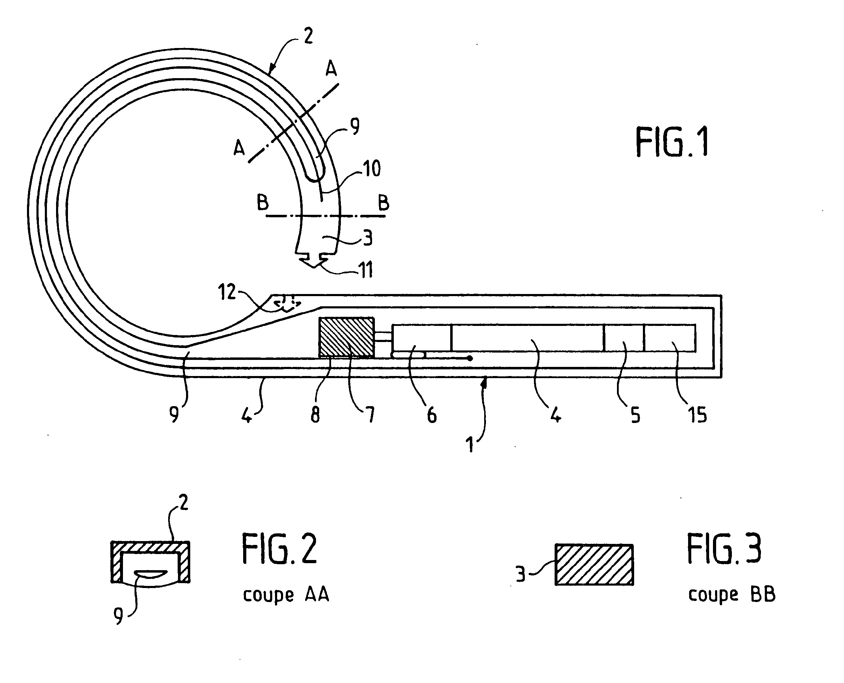

- la figure 1 représente une vue en coupe du dispositif selon l'invention ;
- la figure 2 représente une vue selon un plan de coupe AA de la bande de cerclage.
- la figure 3 représente une vue selon un plan de coupe BB de l'extrémité libre de la bande de cerclage.

Le dispositif de bande gastrique selon l'invention comporte un corps (1) prolongé par une bande de cerclage (2).

La bande de cerclage (2) est formée par un corps creux en silicone, présentant une extrémité libre (3) et une extrémité opposée solidaire du corps (1).

Le corps creux présente une longueur d'environ 60 millimètres et une section d'environ 10 millimètres. Il contient un moteur électrique (4) et son alimentation électrique (15), ainsi qu'un récepteur (5) haute fréquence.

Le moteur (4) entraîne un réducteur (6) dont la sortie entraîne une vis sans fin (7). Cette vis sans fin assure le déplacement de l'extrémité (8) d'un ruban (9). Ce ruban comporte une extrémité (8) présentant un crantage propre à coopérer avec la vis sans fin (8), et une extrémité (10) solidaire de l'extrémité libre (3) de la bande de cerclage.

Cette extrémité libre (3) présente un système d'ancrage (11) en forme de harpon, propre à coopérer avec un système d'accrochage complémentaire (12) prévu sur le corps (1).

Le dispositif se met en place par voie laparoscopique. La bande (2) est mise en place autour de l'estomac, et est ensuite refermée par enclenchement de l'extrémité libre (3) dans le système d'accrochage (12).

Le serrage de la boucle s'effectue par télécommande, en réglant la position de la vis sans fin par une action du moteur électrique télécommandé.

## Revendications

1. - Dispositif de bande gastrique pour former une ouverture restreinte de stoma dans l'estomac de façon à restreindre l'admission de la nourriture vers une partie digestive inférieure de celui-ci, ledit dispositif comprenant un corps (1) prolongé par un élément de bande de cerclage (2) formée en un matériau creux et incluant une portion de bande pour encercler l'estomac de façon à former l'ouverture de stoma restreinte, la portion de bande (2) ayant une extrémité libre (3), ledit corps (1) contenant un moyen motorisé et la bande de cerclage (2) étant entraînée par un moyen télécommandé, **caractérisé en ce que** l'extrémité libre (3) est pourvue d'un moyen de coopération avec ledit corps (1) contenant le moyen motorisé et **en ce que** à l'intérieur de la bande de cerclage (2) coulisse un tendeur dont l'une des extrémités est solidaire de l'extrémité libre de la bande de cerclage (2) et dont l'autre extrémité est entraînée longitudinalement par le moyen motorisé.

2. - Dispositif de bande gastrique pour former une ouverture restreinte de stoma dans l'estomac selon la revendication 1 **caractérisé en ce que** le moyen motorisé comporte une vis sans fin entraînant l'extrémité du tendeur.

3. - Dispositif de bande gastrique pour former une ouverture restreinte de stoma dans l'estomac selon la revendication 1 ou 2 **caractérisé en ce que** le moyen motorisé comporte un moteur électrique télécommandé.

4. - Dispositif de bande gastrique pour former une ouverture restreinte de stoma dans l'estomac selon la revendication 2 ou 3, **caractérisé en ce que** le tendeur est formé par un ruban (9) en un matériau élastique.

5. - Dispositif de bande gastrique pour former une ouverture restreinte de stoma dans l'estomac selon l'une quelconque des revendications 1 à 4 **caractérisé en ce que** le moyen de coopération est formé par un système d'ancrage dans une cavité complémentaire prévue sur le corps contenant le moyen motorisé.

## Claims

1. Gastric banding device for forming a restricted stoma opening in the stomach so as to restrict food intake to a lower digestive portion thereof, said device comprising a body (1) extended by an encircling band portion (2) made from a hollow material and including a band portion for encircling the stomach so as to form the restricted stoma opening, the band portion (2) having one free end (3), said body (1) containing motor means and the encircling band (2) being driven by remote control means, **characterised in that** the free end (3) is equipped with means for cooperating with said body (1) containing the motor means and **in that** a tensioner in which one of the ends is solidly attached to the free end of the encircling band (2) and the other end is driven longitudinally by the motor means slides inside the encircling band (2).

2. Gastric band device for forming a restricted stoma opening in the stomach according to claim 1, **characterised in that** the motor means comprise an endless screw driving the end of the tensioner.

3. Gastric band device for forming a restricted stoma opening in the stomach according to claim 1 or 2, **characterised in that** the motor means include a remote controlled electric motor.

4. Gastric band device for forming a restricted stoma opening in the stomach according to claim 2 or 3, **characterised in that** the tensioner is made up of an elastic band (9).

5. Gastric band device for forming a restricted stoma opening in the stomach according to any one of the claims from 1 to 4, **characterised in that** the cooperation means consist of a system for anchoring in a complementary cavity made in the body containing the motor means.

## Patentansprüche

1. - Vorrichtung eines Magenbands für die Bildung einer eingeschränkten Stomaöffnung im Magen, damit der Einlaß der Nahrung zu einem unteren Verdauungsteil des letzteren eingeschränkt wird, wobei die besagte Vorrichtung einen Körper (1) umfaßt, der durch einen Abschnitt eines Umschnürungsbands (2) verlängert wird, das aus einem hohlen Material gebildet ist und einen Bandabschnitt für das Umschnüren des Magens einschließt, damit die beschränkte Stomaöffnung gebildet wird, wobei der Bandabschnitt (2) ein freies Ende (3) hat, der besagte Körper (1) ein motorbetriebenes Mittel enthält, und das Umschnürungsband (2) durch ein ferngesteuertes Mittel mitgeführt wird, **dadurch gekennzeichnet, daß** das freie Ende (3) mit einem Mittel zur Zusammenarbeit mit dem besagten Körper (1) versehen ist, der das motorbetriebene Mittel enthält, und daß ein Spannglied, von dem eines der Enden fest am freien Ende des Umschnürungsbands (2) befestigt ist, und das andere Ende vom motorbetriebenen Mittel in Längsrichtung mitgeführt wird.

2. - Vorrichtung eines Magenbands für die Bildung einer eingeschränkten Stomaöffnung im Magen nach Anspruch 1, **dadurch gekennzeichnet, daß** das motorbetriebene Mittel eine Schnecke umfaßt, die das Ende des Spannglieds mitführt.

3. - Vorrichtung eines Magenbands für die Bildung einer eingeschränkten Stomaöffnung im Magen nach einem beliebigen der vorstehenden Ansprüche 1 oder 2, **dadurch gekennzeichnet, daß** das motorbetriebene Mittel einen ferngesteuerten Elektromotor enthält.

4. - Vorrichtung eines Magenbands für die Bildung einer eingeschränkten Öffnung des Stomas im Magen, nach einem beliebigen der vorstehenden Ansprüche 2 oder 3, **dadurch gekennzeichnet, daß** das Spannglied aus einem Band (9) aus einem elastischen Material gebildet ist.

5. - Vorrichtung eines Magenbands für die Bildung einer eingeschränkten Stomaöffnung im Magen nach einem beliebigen der vorstehenden Ansprüche 1 bis 4, **dadurch gekennzeichnet, daß** das Zusammenarbeitsmittel durch ein System mit Verankerung in einer zusätzlichen Austiefung gebildet wird, die für den Körper vorgesehen ist, der das motorangetriebene Mittel enthält.
